(19) Europäisches Patentamt / European Patent Office / Office européen des brevets

(11) **EP 4 371 567 A1**

(12) **EUROPEAN PATENT APPLICATION**
published in accordance with Art. 153(4) EPC

(43) Date of publication:
**22.05.2024 Bulletin 2024/21**

(21) Application number: **22841878.6**

(22) Date of filing: **20.06.2022**

(51) International Patent Classification (IPC):
*A61K 38/46* (2006.01)    *A23L 33/195* (2016.01)
*A61K 35/66* (2015.01)    *A61P 1/00* (2006.01)

(52) Cooperative Patent Classification (CPC):
**A23L 33/195; A61K 35/66; A61K 38/46; A61P 1/00**

(86) International application number:
**PCT/JP2022/024561**

(87) International publication number:
**WO 2023/286534 (19.01.2023 Gazette 2023/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.07.2021  JP 2021116710**

(71) Applicants:
• **Amano Enzyme Inc.
Nagoya-shi
Aichi 460-8630 (JP)**
• **Hiroshima University
Higashihiroshima-shi
Hiroshima 739-8511 (JP)**
• **Keio University
Tokyo, 108-8345 (JP)**

(72) Inventors:
• **KURODA, Manabu
Kakamigahara-shi, Gifu 509-0109 (JP)**
• **YAMAGUCHI, Shotaro
Kakamigahara-shi, Gifu 509-0109 (JP)**
• **KATO, Norihisa
Higashihiroshima-shi, Hiroshima 739-8511 (JP)**
• **KUMRUNGSEE, Thanutchaporn
Higashihiroshima-shi, Hiroshima 739-8511 (JP)**
• **YANG, Yongshou
Higashihiroshima-shi, Hiroshima 739-8511 (JP)**
• **FUKUDA, Shinji
Tsuruoka-shi, Yamagata 997-0035 (JP)**

(74) Representative: **Müller-Boré & Partner
Patentanwälte PartG mbB
Friedenheimer Brücke 21
80639 München (DE)**

Remarks:
The complete document including Reference
Table(s) and the Sequence Listing(s) can be
downloaded from the EPO website

(54) **AGENT FOR IMPROVING INTESTINAL BACTERIAL FLORA**

(57)    An object of the present invention is to provide an agent for improving intestinal flora that can increase the numbers of bacteria of genus *Bifidobacterium* and bacteria of genus *Lactobacillus* in the intestine to improve the intestinal flora. (i) A lipase derived from a koji mold and/or (ii) a cellulase can increase the numbers of both bacteria of genus *Bifidobacterium* and bacteria of genus *Lactobacillus* in the intestine to exert an excellent effect of improving the intestinal flora.

EP 4 371 567 A1

**Description**

Technical Field

The present invention relates to an agent for improving intestinal flora that can improve the intestinal flora. More specifically, the present invention relates to an agent for improving intestinal flora that can increase the numbers of both bacteria of genus *Bifidobacterium* and bacteria of genus *Lactobacillus* in the intestine.

Background Art

In recent years, causal relationships between the intestinal environment and various diseases have been intensively investigated to reveal that improvement of the intestinal environment is effective in preventing or ameliorating various diseases. In the intestine, beneficial bacteria, such as bacteria of genus *Bifidobacterium* and bacteria of genus *Lactobacillus,* and bad bacteria, such as *Escherichia coli,* are present in a mixed manner. Forming a beneficial bacteria-dominated flora is important to form a healthy intestinal environment.

Probiotics, prebiotics, and the like have been developed in the past, and various materials by which beneficial bacteria become predominant to improve the intestinal flora have been proposed. It has also been reported that administration of enzymes can improve the intestinal flora.

For example, Non-Patent Literature 1 has reported that the intestinal flora can be improved in mice administered a lipase derived from *Candida rugose.* However, Non-Patent Literature 1 does not disclose that the lipase can increase the numbers of bacteria of genus *Bifidobacterium* and bacteria of genus *Lactobacillus* in the intestine. Thus, no lipases have been found that can increase the numbers of both bacteria of genus *Bifidobacterium* and bacteria of genus *Lactobacillus* in the intestine.

Non-Patent Literature 2 has also reported that in a pig fed with a specific enzyme blend consisting of an amylase, a protease, and a xylanase together with animal feed, the number of lactobacilli is increased and the number of *Escherichia coli* is decreased in the large intestine. Non-Patent Literature 3 has also reported that in a pig fed with a specific enzyme blend (Nopcozyme II; Diasham Resources Pte Ltd.) consisting of an amylase from *Bacillus amyloliquefaciens,* a protease from *Bacillus subtilis,* and a xylanase from *Trichoderma* together with animal feed, the number of lactobacilli is increased and the numbers of *Salmonella* and *Escherichia coli* are decreased. However, it has not been reported that celluloses have the effect of improving the intestinal flora.

Citation List

Non-Patent Literature

> Non-Patent Literature 1: Menden et al., Biomedicine & Pharmacotherapy, 2020, 130: 110579
> Non-Patent Literature 2: Yi et al., Asian Astralas. J. Anim. Sci., 2013, 26: 1181-1188
> Non-Patent Literature 3: Zhang et al., J. Amin. Sci., 2014, 92: 2063-2069

Summary of Invention

Technical Problem

In recent years, in response to the increasing interest in health promotion, the development of new materials by which beneficial bacteria such as bacteria of genus *Bifidobacterium* and bacteria of genus *Lactobacillus* become predominant to improve the intestinal flora has been desired.

It is thus an object of the present invention to provide an agent for improving intestinal flora that can increase the numbers of bacteria of genus *Bifidobacterium* and bacteria of genus *Lactobacillus* in the intestine to improve the intestinal flora.

Solution to Problem

The present inventors have made extensive research to solve the foregoing problem and found that (i) a lipase derived from a koji mold and (ii) a cellulase can increase the numbers of both bacteria of genus *Bifidobacterium* and bacteria of genus *Lactobacillus* in the intestine to exert an excellent effect of improving the intestinal flora. The present inventors have made further research based on the findings, leading to the completion of the invention.

Thus, the present invention includes the following aspects.

Item 1. An agent for improving intestinal flora comprising (i) a lipase derived from a koji mold and/or (ii) a cellulase, as an active ingredient.

Item 2. The agent for improving intestinal flora according to Item 1, wherein the active ingredient is a lipase derived from *Aspergillus niger.*

Item 3. The agent for improving intestinal flora according to Item 1, wherein the active ingredient is a cellulase derived from a koji mold.

Item 4. The agent for improving intestinal flora according to Item 3, wherein the cellulase derived from a koji mold is a cellulase from *Aspergillus niger.*

Item 5. The agent for improving intestinal flora according to any one of Items 1 to 4, wherein the agent is used for increasing the numbers of bacteria of genus *Bifidobacterium* and bacteria of genus *Lactobacillus* in the intestine.

Item 6. A pharmaceutical preparation for oral administration for improving intestinal flora, comprising the agent for improving intestinal flora according to any one of Items 1 to 5.

Item 7. A food additive for improving intestinal flora, comprising the agent for improving intestinal flora according to any one of Items 1 to 5.

Item 8. A food or drink for improving intestinal flora, comprising the agent for improving intestinal flora according to any one of Items 1 to 5.

Item 9. A method for improving intestinal flora comprising orally applying (i) a lipase derived from a koji mold and/or (ii) a cellulase to an individual in need of improvement of the intestinal flora.

Item 10. A lipase derived from a koji mold and/or (ii) a cellulase for use in treatment to improve intestinal flora.

Item 11. Use of a lipase derived from a koji mold and/or (ii) a cellulase for the manufacture of an agent for improving intestinal flora.

Advantageous Effects of Invention

The present invention uses (i) a lipase derived from a koji mold and/or (ii) a cellulase, which can increase the numbers of bacteria of genus *Bifidobacterium* and bacteria of genus *Lactobacillus* in the intestine to improve the intestinal flora, and thus is effective in, for example, forming a healthy intestinal environment, maintaining a healthy intestinal environment, and preventing or treating a disease and/or a symptom due in part to an unhealthy intestinal environment.

Description of Embodiments

An agent for improving intestinal flora of the present invention comprises (i) a lipase derived from a koji mold and/or (ii) a cellulase, as an active ingredient. The agent for improving intestinal flora of the present invention is described in detail below.

[Active Ingredient]

In the agent for improving intestinal flora of the present invention, (i) a lipase derived from a koji mold and/or (ii) a cellulase is used as an active ingredient. By selecting and using (i) the lipase from a koji mold and/or (ii) the cellulase, the numbers of both bacteria of genus *Bifidobacterium* and bacteria of genus *Lactobacillus* in the intestine can be increased to improve the intestinal flora effectively.

In the agent for improving intestinal flora of the present invention, microorganisms from which the lipase is derived are not specifically limited as long as they are koji molds, and include, for example, *Aspergillus niger, Aspergillus oryzae, Aspergillus sojae, Aspergillus saitoi, Aspergillus awamori,* and *Aspergillus flavus.* Of these, a lipase derived from *Aspergillus niger* is suitably used in the present invention, because of its outstanding effect of increasing the numbers of bacteria of genus *Bifidobacterium* and bacteria of genus *Lactobacillus* in the intestine.

In the agent for improving intestinal flora of the present invention, microorganisms from which the cellulase is derived are not specifically limited and include, for example, microorganisms belonging to genus *Aspergillus,* such as *Aspergillus niger, Aspergillus oryzae, Aspergillus sojae, Aspergillus saitoi, Aspergillus awamori,* and *Aspergillus flavus;* microorganisms belonging to genus *Trichoderma,* such as *Trichoderma reesei;* and microorganisms belonging to genus *Acremonium,* such as *Acremonium cellulolyticus.* Of these, a cellulase derived from a microorganism of genus *Aspergillus,* particularly *Aspergillus niger,* is suitably used in the present invention, because of its outstanding effect of increasing the numbers of bacteria of genus *Bifidobacterium* and bacteria of genus *Lactobacillus* in the intestine.

Furthermore, in the agent for improving intestinal flora of the present invention, the type of cellulase used is not specifically limited and may be, for example, an endoglucanase, an exoglucanase, or a mixture thereof. In view of achieving a superior effect of improving the intestinal flora, the cellulase used in the present invention is preferably an enzyme preparation containing both an endoglucanase and an exoglucanase.

(i) the lipase derived from a koji mold and/or (ii) the cellulase used in the present invention may be in a purified form or a partially purified form containing other enzymes and the like.

While (i)the lipase derived from a koji mold and/or (ii) the cellulase can be produced using a known method, such as by microbial culture, these enzymes are commercially available and such commercial products may be used. Examples of commercially available lipases from koji molds include trade names "Lipase AP6" and "Lipase AP12" (lipase preparations derived from *Aspergillus niger;* Amano Enzyme Inc.); trade name "Sumizyme NLS" (a lipase preparation derived from *Aspergillus niger*; Shin Nihon Chemicals Corporation); and trade name Panamore (a lipase preparation derived from *Aspergillus niger*; DSM Japan K.K.). Examples of commercially available cellulases include trade name "Cellulase A 'Amano' 3" (a cellulase preparation derived from *Aspergillus niger*; Amano Enzyme Inc.); trade name "GODO Cellulase F" (a cellulase preparation derived from *Trichoderma reesei*; Godo Shusei Co., LTD.); trade name "BakeZyme X?CEL" (a cellulase preparation derived from *Trichoderma reesei*; DSM Japan K.K.); trade name "Sumizyme AC" (a cellulase preparation derived from *Aspergillus niger*; Shin Nihon Chemicals Corporation); and trade name "Celluzyme C" (a cellulase preparation derived from *Trichoderma* sp.; Nagase ChemteX Corporation). Of these commercial products, "Lipase AP12" and "Cellulase A 'Amano' 3" have an outstanding effect of increasing the numbers of bacteria of genus *Bifidobacterium* and bacteria of genus *Lactobacillus* in the intestine, and thus are suitably used in the present invention.

In the agent for improving intestinal flora of the present invention, one enzyme selected from (i) the lipase derived from koji molds and (ii) the cellulase may be used alone, or two or more enzymes from (i) the lipase derived from koji molds and (ii) the cellulase may be used in combination.

[Dose]

The dose of the agent for improving intestinal flora of the present invention may be determined appropriately according to, for example, the type of active ingredient used, the type of product in which the agent is used, the use, the expected effect, and the form of application.

For example, when a lipase derived from a koji mold is used, the daily dose for an adult human as an amount of the lipase derived from a koji mold ingested or administered may be 1,000 to 100,000 U, preferably 3,000 to 60,000 U, and more preferably 5,000 to 30,000 U. As used herein, the lipase activity (U) is a value determined using the following method:

(Method of Measuring Lipase Activity)

200 to 300 mL of a mixture of a 2 w/v% aqueous polyvinyl alcohol solution and olive oil at a volume ratio of 3:1 is placed in an emulsifier container and emulsified at 12,000 to 16,000 revolutions per minute for 10 minutes while cooling to 10°C or less. After emulsification, the emulsion is left standing in a cool place for 1 hour, and, after confirming that the oil layer does not separate, the emulsion is used as a substrate solution.

First, 5 mL of the substrate solution and 4 mL of 0.1 mol/L phosphate buffer (pH 6.0) are precisely weighed into a flat-bottom test tube (30 × 120 mm), shaken, and left standing at 37 ± 0.5°C for 10 minutes; then, precisely 1 mL of a sample solution containing the sample whose activity is to be measured is added and immediately shaken. This solution is left standing at 37 ± 0.5°C for precisely 20 minutes, and then 10 mL of a 1:1 volume mixture of ethanol and acetone is added and shaken. Then, precisely 10 mL of a 0.05 mol/L aqueous sodium hydroxide solution is added, and additionally 10 mL of a 1:1 volume mixture of ethanol and acetone is added and shaken, and then excess sodium hydroxide is titrated with 0.05 mol/L hydrochloric acid (b mL) (indicator: 2 to 3 drops of phenolphthalein reagent).

Separately, 5 mL of the substrate solution and 4 mL of 0.1 mol/L phosphate buffer (pH 6.0) are each precisely weighed into a flat-bottom test tube (30 × 120 mm), shaken, and left standing at 37 ± 0.5°C for 10 minutes; then, 10 mL of a 1:1 volume mixture of ethanol and acetone is added, and then precisely 1 mL of a sample solution containing the sample whose activity is to be measured is added and shaken. Then, precisely 10 mL of a 0.05 mol/L aqueous sodium hydroxide solution is added and thereafter, the same procedure is repeated, and titration is performed (a mL).

For titration, a pH meter is used, and 0.05 mol/L hydrochloric acid is added until the pH reaches 10.00. Under these conditions, the amount of enzyme that causes an increase in fatty acids by 1 micromole (μmol) per minute is defined as 1 U. The following equation is used for the calculation.

Lipase activity per g or mL of sample (U/g or U/mL) = $50 \times (a - b) \times 1/20 \times f \times 1/M$

a: titer value (mL) of blank
b: titer value (mL) of reaction solution

50: equivalent amount of fatty acids (micromole) per mL of 0.05 mol/L hydrochloric acid

20: reaction time (min)

M: amount of sample (g or mL) in 1 mL of test solution

f: factor of 0.05 mol/L hydrochloric acid

For example, when a cellulase is used, the daily dose for an adult human as an amount of the cellulase ingested or administered may be 2,000 to 200,000 U, preferably 6,000 to 120,000 U, and more preferably 10,000 to 60,000 U. As used herein, the cellulase activity (U) is a value determined using the following method:

(Method of Measuring Cellulase Activity)

First, 4 mL of a 0.5 w/v% aqueous carmellose sodium solution (pH 4.5) is weighed into a 50 mL Nessler tube and left standing at $40 \pm 0.5°C$ for 10 to 15 minutes, and then 1 mL of a sample solution containing the sample whose activity is to be measured is added and immediately shaken.

This solution is left standing at $40 \pm 0.5°C$ for precisely 30 minutes, then 2 mL of the Somogyi reagent (a solution containing 2.4 w/v% anhydrous sodium carbonate, 1.2 w/v% sodium potassium tartrate tetrahydrate, 0.4 w/v% copper(II) sulfate pentahydrate, 1.6 w/v% sodium hydrogen carbonate, and 1.8 w/v% anhydrous sodium sulfate) is added and shaken, the tube is stoppered and heated in a boiling water bath for precisely 20 minutes and then cooled immediately. After cooling, 1 mL of the Nelson reagent is added and shaken well until complete dissolution of a red precipitate of cuprous oxide, and the solution is left standing at room temperature for about 20 minutes, adjusted to a total volume of 25 mL with 17 mL of water, and shaken well.

Absorbance (A30) at a wavelength of 500 nm is measured for the resulting solution, using water as a control. Separately, 2 mL of the Somogyi reagent is added to 4 mL of a 0.5 w/v% aqueous carmellose sodium solution (pH 4.5) and shaken, and then 1 mL of a sample solution containing the sample whose activity is to be measured is added and thereafter, the same procedure is repeated for the resulting solution, and absorbance (A0) is measured.

Also, separately, the same procedure is repeated using a 20 to 60 μg/mL glucose solution, and a glucose standard curve is prepared.

Under these conditions, the amount of enzyme that produces reducing sugars equivalent to 1 mg of glucose per minute is defined as 100 U. The following equation is used for the calculation.

[Expression 2]

$$\text{Cellulase activity per g or mL of sample (U/g or U/mL)} = G \times 1/30 \times 100 \times n$$

G: amount (mg) of produced glucose determined from glucose standard curve, using value (A30 - A0)

1/30: conversion factor into per minute

100: conversion factor into 100 units

n: dilution factor per g or mL of sample

[Use]

The agent for improving intestinal flora of the present invention can increase the number of beneficial bacteria such as bifidobacteria and lactic acid bacteria in the intestine, by the effect of (i) the lipase derived from a koji mold and/or (ii) the cellulase, to form a beneficial bacteria-dominated flora, and thus is used for the purpose of forming a healthy intestinal environment, maintaining a healthy intestinal environment, and the like. In particular, the agent for improving intestinal flora of the present invention has an excellent effect of increasing the numbers of both bacteria of genus *Bifidobacterium* and bacteria of genus *Lactobacillus* in the intestine, and thus can also be used as an agent for increasing the numbers of bacteria of genus *Bifidobacterium* and bacteria of genus *Lactobacillus* in the intestine.

Additionally, the agent for improving intestinal flora of the present invention can form a beneficial bacteria-dominated flora to form a healthy intestinal environment, and thus can also be used for the purpose of preventing or treating a disease and/or a symptom due in part to an unhealthy intestinal environment. Examples of the disease and/or symptom include decreased immunity, colorectal cancer, allergic disease, nonalcoholic steatohepatitis, obesity, and inflammatory bowel disease.

[Form of Use]

The agent for improving intestinal flora of the present invention is orally applied by oral ingestion or oral administration. The agent for improving intestinal flora of the present invention is orally ingested or orally administered to exert the effect of improving intestinal flora after arriving at the intestine, and thus can be blended for use with various products such as foods and drinks, pharmaceutical preparations for oral administration, animal feed, and pet foods.

When the agent for improving intestinal flora of the present invention is blended with the various products, the products may contain probiotics and/or prebiotics as required together with the agent for improving intestinal flora of the present invention.

Examples of microorganisms used as the probiotics include lactic acid bacteria, bifidobacteria, and *Bacillus subtilis var natto.* Specific examples of the lactic acid bacteria include lactic acid bacteria of genus *Lactobacillus,* such as *Lactobacillus casei, Lactobacillus acidophilus,* and *Lactobacillus plantarum;* lactic acid bacteria of genus *Enterococcus,* such as *Enterococcus faecalis, Enterococcus faecium,* and *Enterococcus hirae;* and lactic acid bacteria of genus *Streptococcus,* such as *Streptococcus bovis* and *Streptococcus thermophilus.* Specific examples of the bifidobacteria include *Bifidobacterium adolescentis, Bifidobacterium longum, Bifidobacterium bifidum, Bifidobacterium pseudolongum,* and *Bifidobacterium thermophilum.* These probiotics may be used alone or in combination.

Examples of the prebiotics include xylooligosaccharides, fructooligosaccharides, soybean oligosaccharides, isomaltooligosaccharides, lacto-fructo-oligosaccharides, galactooligosaccharides, and lactulose. These prebiotics may be used alone or in combination.

The dosage form of a product with which the agent for improving intestinal flora of the present invention is blended may be any of a solid form, a semi-solid form, a liquid form, and the like, and is selected appropriately according to the type and use of the product.

When the agent for improving intestinal flora of the present invention is used for foods and drinks, (i) the lipase derived from a koji mold and/or (ii) the cellulase is provided as a food or a drink that exerts the effect of improving intestinal flora, either as is or in combination with other food materials or additive ingredients to be prepared in a desired form. Examples of the food and drink include foods for specified health uses, nutritional supplements, functional foods, and foods for patients, as well as common foods and drinks. Forms of these foods and drinks include, but are not limited to, supplements, such as tablets, granules, powders, capsules, and soft capsules; and drinks, such as energy drinks, fruit drinks, carbonated beverages, and lactic acid drinks.

When the agent for improving intestinal flora of the present invention is used for foods and drinks, the amount of the agent blended into the food and drink may be determined appropriately according to, for example, the type of active ingredient used, and the form of the food and drink, within a range satisfying the above-described dose.

For example, when the lipase derived from a koji mold is used as the agent for improving intestinal flora of the present invention and is provided in the form of a supplement, the amount of the lipase derived from a koji mold blended into the supplement may be 300 to 50,000 U/g, preferably 1,000 to 30,000 U/g, and more preferably 1,500 to 15,000 U/g.

For example, when the lipase derived from a koji mold is used as the agent for improving intestinal flora of the present invention and is provided in the form of a drink, the amount of the lipase derived from a koji mold blended into the drink may be 3 to 500 U/mL, preferably 10 to 300 U/mL, and more preferably 15 to 150 U/mL.

For example, when the cellulase is used as the agent for improving intestinal flora of the present invention and is provided in the form of a supplement, the amount of the cellulase blended into the supplement may be 600 to 100,000 U/g, preferably 2,000 to 60,000 U/g, and more preferably 3,000 to 30,000 U/g.

For example, when the cellulase is used as the agent for improving intestinal flora of the present invention and is provided in the form of a drink, the amount of the cellulase blended into the drink may be 6 to 1,000 U/mL, preferably 20 to 600 U/mL, and more preferably 30 to 300 U/mL.

When the agent for improving intestinal flora of the present invention is used in the field of foods and drinks, (i) the lipase derived from a koji mold and/or (ii) the cellulase can be provided as a food additive for improving intestinal flora, either alone or in combination with other ingredients.

When the agent for improving intestinal flora of the present invention is used for a pharmaceutical preparation for oral administration, the agent for improving intestinal flora of the present invention is provided as a pharmaceutical preparation for oral administration that exert the effect of improving intestinal flora, either alone or in combination with, for example, other pharmacologically active ingredients, pharmaceutically acceptable bases, or additives to be prepared in a desired form. Forms of the pharmaceutical preparation specifically include, but are not limited to, preparations for oral administration, such as tablets, granules, powders, capsules, soft capsules, syrups, and liquids.

Examples of the bases and additives used for manufacturing the pharmaceutical preparation for oral administration include aqueous bases such as water and alcohols, oily bases, excipients, binders, filling agents, disintegrants, lubricants, algefacients, pH-adjusting agents, thickeners, antioxidants, sequestering agents, surfactants, emulsifiers, solubilizers, solubilizing agents, flavoring agents, and antiseptics.

When the agent for improving intestinal flora of the present invention is used as a pharmaceutical preparation for oral administration, the proportion of the agent blended into the pharmaceutical preparation for oral administration may be determined appropriately according to, for example, the type of active ingredient used and the form of the pharmaceutical preparation for oral administration, within a range satisfying the above-described dose.

**EP 4 371 567 A1**

For example, when the lipase derived from a koji mold is used as the agent for improving intestinal flora of the present invention and is provided in the form of a solid or semi-solid pharmaceutical preparation for oral administration, the amount of the lipase derived from a koji mold blended into the solid or semi-solid pharmaceutical preparation for oral administration may be 300 to 50,000 U/g, preferably 1,000 to 30,000 U/g, and more preferably 1,500 to 15,000 U/g.

For example, when the lipase derived from a koji mold is used as the agent for improving intestinal flora of the present invention and is provided in the form of a liquid pharmaceutical preparation for oral administration, the amount of the lipase derived from a koji mold blended into the liquid pharmaceutical preparation for oral administration may be 3 to 500 U/mL, preferably 10 to 300 U/mL, and more preferably 15 to 150 U/mL.

For example, when the cellulase is used as the agent for improving intestinal flora of the present invention and is provided in the form of a solid or semi-solid pharmaceutical preparation for oral administration, the amount of the cellulase blended into the solid or semi-solid pharmaceutical preparation for oral administration may be 600 to 100,000 U/g, preferably 2,000 to 60,000 U/g, and more preferably 3,000 to 30,000 U/g.

For example, when the cellulase is used as the agent for improving intestinal flora of the present invention and is provided in the form of a liquid pharmaceutical preparation for oral administration, the amount of the cellulase blended into the liquid pharmaceutical preparation for oral administration may be 6 to 1,000 U/mL, preferably 20 to 600 U/mL, and more preferably 30 to 300 U/mL.

When the agent for improving intestinal flora of the present invention is used for animal feed or a pet food, the agent for improving intestinal flora of the present invention is provided as animal feed or a pet food that exerts the effect of improving intestinal flora, either alone or in combination with other animal feed ingredients to be prepared in a desired form. Examples of the animal feed ingredients used for the animal feed or pet food include cereal crops, such as corn, wheat, barley, and rye; bran, such as wheat bran and rice bran; dregs, such as corn gluten meal and corn germ meal; animal-derived feed, such as skimmed milk powder, whey, fish flour, and powdered bone; yeasts, such as brewer's yeast; calcium, such as calcium phosphate and calcium carbonate; vitamins; amino acids; and saccharides.

When the agent for improving intestinal flora of the present invention is used as animal feed or a pet food, the proportion of the agent blended into the animal feed or pet food may be determined appropriately according to, for example, the form and type of the animal feed or pet food, and the type of the animal to which the agent is applied.

For example, when the lipase derived from a koji mold is used, the amount of the lipase derived from a koji mold blended into the animal feed or pet food may be 2 to 200 U/g, preferably 6 to 120 U/g, and more preferably 10 to 60 U/g.

For example, when the cellulase is used, the amount of the cellulase blended into the animal feed or pet food may be 4 to 400 U/g, preferably 12 to 240 U/g, and more preferably 20 to 120 U/g.

Examples

The present invention is described more specifically below with reference to examples, although the present invention should not be construed to be limited to these examples.

Test Example 1: Influence of Lipase derived from *Aspergillus niger* on Intestinal Flora

Using SD rats (male, 4 weeks old, from Hiroshima Institute for Experimental Animals), influence of the ingestion of Lipase AP12 (Amano Enzyme Inc.) (lipase activity: 13.5 U/mg), which is a lipase preparation from *Aspergillus niger,* on the intestinal flora was investigated. In the experiment, the rats were divided into two groups of a control group and a working group, with eight rats per group. The rats of each group were fed *ad libitum* for 14 days with the animal feed shown in Table 1.

[Table 1]

|  | Control | Example 1 |
|---|---|---|
| Lipase AP12 | - | 0.1 |
| Beef tallow | 30.0 | 30.0 |
| Casein | 20.0 | 20.0 |
| L-Cystine | 0.3 | 0.3 |
| Vitamin Mix# | 1.0 | 1.0 |
| Mineral Mix# | 3.5 | 3.5 |
| Cellulose | 5.0 | 5.0 |

(continued)

|  | Control | Example 1 |
|---|---|---|
| Sucrose | 20.0 | 20.0 |
| Corn starch | 20.2 | 20.1 |
| Total | 100.0 | 100.0 |
| The content of each ingredient in the table is in "% by weight". * refers to AIN-93 (a standard purified diet composition for mice and rats formulated by the American Institute of Nutrition (AIN)). | | |

After 14 days of feeding with the animal feed, the body weights and the weights of cecal contents of the rats in each group were determined, and, after extraction of DNA from the cecal contents, quantitative PCR analysis (Yang et al. Biomed Rep 2015, 3: 715-720) was performed to quantify the copy numbers (numbers of bacteria) of bacteria of genus *Bifidobacterium* (*Bifidobacterium* spp.) and bacteria of genus *Lactobacillus* (*Lactobacillus* spp.) in the cecal contents. In this PCR analysis, the bacterial species-specific primers shown in Table 2 were used. Additionally, 16S rRNA gene sequencing analysis (Mishima et al. Am J Physiol Renal Physiol, 2018, 315: F824-F833) was performed to quantify the numbers of bacteria of genus *Bifidobacterium* and bacteria of genus *Lactobacillus* in the cecal contents.

[Table 2]

|  | Nucleotide sequence of the primer used |
|---|---|
| For detection of *Bifidobacterium* spp. | Forward : CGCGTCYGGTGTGAAAG |
|  | Reverse: CCCCACATCCAGCATCCA |
| For detection of *Lactobacillus* spp. | Forward : GAGGCAGCAGTAGGGAATCTTC |
|  | Reverse : GGCCAGTTACTACCTCTATCCTTCTTC |

The results are shown in Tables 3 and 4. In Example 1, the numbers of bacteria of genus *Bifidobacterium* and bacteria of genus *Lactobacillus* in the intestine were both higher than those in the control. That is, it was revealed that ingestion of the lipase derived from *Aspergillus niger* promoted the proliferation of bacteria of genus *Bifidobacterium* and bacteria of genus *Lactobacillus* in the intestine, leading to improvement of the intestinal flora.

[Table 3]

| Average body weight, weight of cecal contents and results of quantitative PCR analysis | | | |
|---|---|---|---|
|  |  | Control | Example 1 |
| Average body weight (g) | | $211\pm3$ | $213\pm3$ |
| Weight (g) of cecal contents | | $1.44\pm0.06$ | $4.72\pm0.39^*$ |
| Bacteria of genus *Bifidobacterium* | Copy number ($\times10^6$ / g contents) | $2.4\pm0.7$ | $62.8\pm12.5^*$ |
|  | Copy number ($\times10^6$ / total contents) | $3.5\pm1.1$ | $278.1+44.5^*$ |
| Bacteria of genus *Lactobacillus* | Copy number ($\times10^6$ / g contents) | $2.77\pm0.78$ | $7.56\pm2.36$ |
|  | Copy number ($\times10^6$ / total contents) | $3.8\pm1.08$ | $38.2\pm13.8^*$ |
| The measured results in the table are expressed as mean $\pm$ standard error (SE), and * indicates $p < 0.05$ by Student's t test. | | | |

[Table 4]

| Results of 16S rRNA sequencing analysis | | |
|---|---|---|
|  | Control | Example 1 |
| Bacteria of genus *Bifidobacterium* (% of total bacteria) | $0.34\pm0.11$ | $40.76\pm4.89^*$ |

(continued)

| Results of 16S rRNA sequencing analysis | | |
|---|---|---|
| | Control | Example 1 |
| Bacteria of genus *Lactobacillus* (% of total bacteria) | 6.98±1.16 | 24.00±6.82* |
| The measured results in the table are expressed as mean ± standard error (SE), and * indicates p < 0.05 by Student's t test. | | |

Test Example 2: Influence of Cellulase derived from *Aspergillus niger* on Intestinal Flora

Using SD rats (male, 4 weeks old, from Hiroshima Institute for Experimental Animals), influence of the ingestion of Cellulase A "Amano" 3 (Amano Enzyme Inc.) (cellulase activity: 33 U/mg), which is a cellulase preparation derived from *Aspergillus niger* (a cellulase preparation exhibiting both endoglucanase activity and exoglucanase activity), on the intestinal flora was investigated. In the experiment, the rats were divided into two groups of a control group and a working group, with six to eight rats per group. The rats of each group were fed *ad libitum* for 14 days with the animal feed shown in Table 5.

[Table 5]

| | Control | Example 2 |
|---|---|---|
| Cellulase A "Amano" 3 | - | 0.1 |
| Beef tallow | 30.0 | 30.0 |
| Casein | 20.0 | 20.0 |
| L-Cystine | 0.3 | 0.3 |
| Vitamin Mix# | 1.0 | 1.0 |
| Mineral Mix# | 3.5 | 3.5 |
| Cellulose | 5.0 | 5.0 |
| Sucrose | 20.0 | 20.0 |
| Corn starch | 20.2 | 20.1 |
| Total | 100.0 | 100.0 |
| The content of each ingredient in the table is in "% by weight". # refers to AIN-93 (a standard purified diet composition for mice and rats formulated by the American Institute of Nutrition (AIN)). | | |

After 14 days of feeding with the animal feed, the body weights and the weights of cecal contents of the rats in each group were determined, and quantitative PCR analysis and 16S rRNA gene sequencing analysis were performed using the same methods as in Test Example 1 above to quantify the numbers of bacteria of genus *Bifidobacterium* and bacteria of genus *Lactobacillus* in the cecal contents.

The results are shown in Tables 6 and 7. In Example 2, the numbers of bacteria of genus *Bifidobacterium* and bacteria of genus *Lactobacillus* in the intestine were both higher than those in the control. That is, it was confirmed that ingestion of the cellulase derived from *Aspergillus niger* promoted the proliferation of bacteria of genus *Bifidobacterium* and bacteria of genus *Lactobacillus* in the intestine, leading to improvement of the intestinal flora.

[Table 6]

| Average body weight, weight of cecal contents and results of quantitative PCR analysis | | |
|---|---|---|
| | Control | Example 2 |
| Average body weight (g) | 211±3 | 214±2 |
| Weight (g) of cecal contents | 1.44±0.06 | 5.05±0.31* |

(continued)

| Average body weight, weight of cecal contents and results of quantitative PCR analysis | | | |
|---|---|---|---|
| | | Control | Example 2 |
| Bacteria of genus *Bifidobacterium* | Copy number ($\times 10^6$ 1 g contents) | $2.4 \pm 0.7$ | $5.59 \pm 1.59$ |
| | Copy number $\times 10^6$ / total contents) | $3.5 \pm 1.1$ | $28.5 \pm 9.2^*$ |
| Bacteria of genus *Lactobacillus* | Copy number ($\times 10^6$ / g contents) | $2.77 \pm 0.78$ | $13.92 \pm 4.44^*$ |
| | Copy number ($\times 10^6$ / total contents) | $3.8 \pm 1.08$ | $78.1 + 34.5^*$ |
| The measured results in the table are expressed as mean $\pm$ standard error (SE), and * indicates $p < 0.05$ by Student's t test. | | | |

[Table 7]

| Results of 16S rRNA analysis | | |
|---|---|---|
| | Control | Example 2 |
| Bacteria of genus *Bifidobacterium* (% of total bacteria) | $0.34 \pm 0.11$ | $9.65 \pm 2.12^*$ |
| Bacteria of genus *Lactobacillus* (% of total bacteria) | $6.98 \pm 1.16$ | $31.86 \pm 8.91^*$ |
| The measured results in the table are expressed as mean $\pm$ standard error (SE), and * indicates $p < 0.05$ by Student's t test. | | |

**Claims**

1. An agent for improving intestinal flora comprising (i) a lipase derived from a koji mold and/or (ii) a cellulase, as an active ingredient.

2. The agent for improving intestinal flora according to claim 1, wherein the active ingredient is a lipase derived from *Aspergillus niger.*

3. The agent for improving intestinal flora according to claim 1, wherein the active ingredient is a cellulase derived from a koji mold.

4. The agent for improving intestinal flora according to claim 3, wherein the cellulase derived from a koji mold is a cellulase derived from *Aspergillus niger.*

5. The agent for improving intestinal flora according to claim 1 or 2, wherein the agent is used for increasing the numbers of bacteria of genus *Bifidobacterium* and bacteria of genus *Lactobacillus* in the intestine.

6. A pharmaceutical preparation for oral administration for improving intestinal flora, comprising the agent for improving intestinal flora according to claim 1 or 2.

7. A food additive for improving intestinal flora, comprising the agent for improving intestinal flora according to claim 1 or 2.

8. A food or drink for improving intestinal flora, comprising the agent for improving intestinal flora according to claim 1 or 2.

9. A method for improving intestinal flora comprising orally applying (i) a lipase derived from a koji mold and/or (ii) a cellulase to an individual in need of improvement of the intestinal flora.

10. Use of (i) a lipase derived from a koji mold and/or (ii) a cellulase for the manufacture of an agent for improving intestinal flora.

**11.** (i) A lipase derived from a koji mold and/or (ii) a cellulase for use in treatment to improve intestinal flora.

| INTERNATIONAL SEARCH REPORT | International application No. |
| --- | --- |
| | **PCT/JP2022/024561** |

**A. CLASSIFICATION OF SUBJECT MATTER**

*A61K 38/46*(2006.01)i; *A23L 33/195*(2016.01)i; *A61K 35/66*(2015.01)i; *A61P 1/00*(2006.01)i
FI:  A61K38/46; A61P1/00; A61K35/66; A23L33/195

According to International Patent Classification (IPC) or to both national classification and IPC

**B. FIELDS SEARCHED**

Minimum documentation searched (classification system followed by classification symbols)

A61K38/46; A23L33/195; A61K35/66; A61P1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2016-102097 A (GEORGE & OLIVER CO LTD) 02 June 2016 (2016-06-02) | 1, 3, 5-11 |
| | claims 1, 3, 5, paragraph [0031], examples, table 4 | |
| Y | | 2, 4 |
| X | WO 2007/034627 A1 (IDEMITSU KOSAN CO., LTD.) 29 March 2007 (2007-03-29) | 1-4, 6, 9-11 |
| | claim 1, paragraphs [0027], [0030], examples, table 16 | |
| Y | | 2, 4 |
| A | JP 2000-26311 A (AMANO PHARMACEUT CO LTD) 25 January 2000 (2000-01-25) | 1-11 |
| | claim 1, examples | |
| P, X | YANG, Y. et al. Supplemental Aspergillus Lipase and Protease Preparations Display Powerful Bifidogenic Effects and Modulate the Gut Microbiota Community of Rats. Fermentation. 01 December 2021, 7, 294, pp. 1-13, https://doi.org/10.3390/fermentation7040294 | 1, 2, 5-11 |
| | abstract | |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 July 2022** | **26 July 2022** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**

| International application No. |
| --- |
| **PCT/JP2022/024561** |

**C. DOCUMENTS CONSIDERED TO BE RELEVANT**

| Category* | Citation of document, with indication, where appropriate, of the relevant passages | Relevant to claim No. |
| --- | --- | --- |
| P, X | YANG, Y. et al. Aspergillus-Derived Cellulase Preparation Exhibits Prebiotic-like Effects on Gut Microbiota in Rats. Fermentation. 08 February 2022, 8, 71, pp. 1-12, https://doi.org/10.3390/fermentation8020071<br>abstract | 1, 3-11 |

Form PCT/ISA/210 (second sheet) (January 2015)

**INTERNATIONAL SEARCH REPORT**
Information on patent family members

International application No.

**PCT/JP2022/024561**

| Patent document cited in search report | | | Publication date (day/month/year) | Patent family member(s) | Publication date (day/month/year) |
|---|---|---|---|---|---|
| JP | 2016-102097 | A | 02 June 2016 | (Family: none) | |
| WO | 2007/034627 | A1 | 29 March 2007 | US 2009/0155417 A1 claim 1, paragraphs [0044], [0047], examples, table 16 | |
| JP | 2000-26311 | A | 25 January 2000 | US 2002/0018773 A1 claim 1, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MENDEN et al.** *Biomedicine & Pharmacotherapy,* 2020, vol. 130, 110579
- **YI ; ASIAN ASTRALAS. et al.** *J. Anim. Sci.,* 2013, vol. 26, 1181-1188
- **ZHANG et al.** *J. Amin. Sci.,* 2014, vol. 92, 2063-2069
- **YANG et al.** *Biomed Rep,* 2015, vol. 3, 715-720
- **MISHIMA et al.** *Am J Physiol Renal Physiol,* 2018, vol. 315, F824-F833